(19) Europäisches Patentamt / European Patent Office / Office européen des brevets

(11) **EP 4 027 348 A2**

(12) **EUROPEAN PATENT APPLICATION**

(43) Date of publication:
**13.07.2022 Bulletin 2022/28**

(21) Application number: **21202323.8**

(22) Date of filing: **13.10.2021**

(51) International Patent Classification (IPC):
**G16B 15/30** (2019.01)    **G16B 40/20** (2019.01)

(52) Cooperative Patent Classification (CPC):
**G16B 15/30; G16B 40/20**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.01.2021 CN 202110011160**

(71) Applicant: **BEIJING BAIDU NETCOM SCIENCE AND TECHNOLOGY CO. LTD.**
**100085 Beijing (CN)**

(72) Inventors:
• **WANG, Fan**
  **Beijing, 100085 (CN)**

• **HE, Jingzhou**
  **Beijing, 100085 (CN)**
• **FANG, Xiaomin**
  **Beijing, 100085 (CN)**
• **ZHANG, Xiaonan**
  **Beijing, 100085 (CN)**
• **WU, Hua**
  **Beijing, 100085 (CN)**
• **WU, Tian**
  **Beijing, 100085 (CN)**
• **WANG, Haifeng**
  **Beijing, 100085 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **AFFINITY PREDICTION METHOD AND APPARATUS, METHOD AND APPARATUS FOR TRAINING AFFINITY PREDICTION MODEL, DEVICE AND MEDIUM**

(57)    The present disclosure discloses an affinity prediction method and apparatus, a method and apparatus for training an affinity prediction model, a device and a medium, and relates to the field of artificial intelligence technologies, such as machine learning technologies, smart medical technologies, or the like. An implementation includes: collecting a plurality of training samples, each training sample including information of a training target, information of a training drug and a test data set corresponding to the training target; and training an affinity prediction model using the plurality of training samples. In addition, there is further disclosed the affinity prediction method. The technology in the present disclosure may effectively improve accuracy and a training effect of the trained affinity prediction model. During an affinity prediction, accuracy of a predicted affinity of a target to be detected with a drug to be detected may be higher by acquiring a test data set corresponding to the target to be detected to participate in the prediction.

Collect a plurality of training samples, each training sample including information of a training target, information of a training drug and a test data set corresponding to the training target — S101

Train an affinity prediction model using the plurality of training samples — S102

Fig. 1

**Description**

**Field of the Disclosure**

The present disclosure relates to the field of computer technologies, and particularly relates to the field of artificial intelligence technologies, such as machine learning technologies, smart medical technologies, or the like, and particularly to an affinity prediction method and apparatus, a method and apparatus for training an affinity prediction model, a device and a medium.

**Background of the Disclosure**

Usually, a target of a human disease is a protein playing a key role in a development of the disease, and may also be referred to as a protein target. A drug makes the corresponding protein lose an original function by binding to the target protein, thereby achieving an inhibition effect on the disease. In a process of researching and developing a new drug, a prediction of an affinity between the protein target and a compound molecule (drug) is a quite important link. With the affinity prediction, a high-activity compound molecule which may be tightly bound to the protein target is found and continuously optimized to finally form the drug available for treatment.

In a conventional method, an in-vitro activity experiment is required to be performed on the compound molecules of the finally formed drug one by one to accurately detect the affinity between the drug and the protein target. Although high throughput experiments may now be performed hundreds or thousands of times in a short time, such an experiment still has a quite high cost, and such an experimental approach is still not feasible in the face of an almost infinite compound space and tens of millions of compound structures.

**Summary of the Disclosure**

The present disclosure provides an affinity prediction method and apparatus, a method and apparatus for training an affinity prediction model, a device and a medium.

According to an aspect of the present disclosure, there is provided a method for training an affinity prediction model, including:

collecting a plurality of training samples, each training sample including information of a training target, information of a training drug and a test data set corresponding to the training target; and
training an affinity prediction model using the plurality of training samples.

According to another aspect of the present disclosure, there is provided an affinity prediction method, including:

acquiring information of a target to be detected, information of a drug to be detected and a test data set corresponding to the target to be detected; and
predicting an affinity between the target to be detected and the drug to be detected using a pre-trained affinity prediction model based on the information of the target to be detected, the information of the drug to be detected and the test data set corresponding to the target to be detected.

According to still another aspect of the present disclosure, there is provided a method for screening drug data, including:

screening information of several drugs with a highest predicted affinity with a preset target from a preset drug library using a pre-trained affinity prediction model based on a test data set corresponding to the preset target;
acquiring a real affinity of each of the several drugs with the preset target obtained by an experiment based on the screened information of the several drugs; and
updating the test data set corresponding to the preset target based on the information of the several drugs and the real affinity of each drug with the preset target.

According to yet another aspect of the present disclosure, there is provided an apparatus for training an affinity prediction model, including:

a collecting module configured to collect a plurality of training samples, each training sample including information of a training target, information of a training drug and a test data set corresponding to the training target; and
a training module configured to train an affinity prediction model using the plurality of training samples.

According to another aspect of the present disclosure, there is provided an affinity prediction apparatus, including:

an acquiring module configured to acquire information of a target to be detected, information of a drug to be detected and a test data set corresponding to the target to be detected; and
a predicting module configured to predict an affinity between the target to be detected and the drug to be detected using a pre-trained affinity prediction model based on the information of the target to be detected, the information of the drug to be detected and the test data set corresponding to the target to be detected.

According to another aspect of the present disclosure, there is provided an apparatus for screening drug data, including:

a screening module configured to screen information of several drugs with a highest predicted affinity with a preset target from a preset drug library using a pre-trained affinity prediction model based on a test data set corresponding to the preset target;
an acquiring module configured to acquire a real affinity of each of the several drugs with the preset target obtained by an experiment based on the screened information of the several drugs; and
an updating module configured to update the test data set corresponding to the preset target based on the information of the several drugs and the real affinity of each drug with the preset target.

According to another aspect of the present disclosure, there is provided an electronic device, including:

at least one processor; and
a memory connected with the at least one processor communicatively;
wherein the memory stores instructions executable by the at least one processor to enable the at least one processor to perform the method as mentioned above.

According to another aspect of the present disclosure, there is provided a non-transitory computer readable storage medium with computer instructions stored thereon, wherein the computer instructions are used for causing a computer to perform the method as mentioned above.

According to another aspect of the present disclosure, there is provided a computer program which, when executed by a processor, implements the method as mentioned above.

According to the technology in the present disclosure, when the affinity prediction model is trained, the test data set corresponding to the training target may be added in each training sample, thus effectively improving accuracy and a training effect of the trained affinity prediction model. During the affinity prediction, the accuracy of the predicted affinity of the target to be detected with the drug to be detected may be higher by acquiring the test data set corresponding to the target to be detected to participate in the prediction.

It should be understood that the statements in this section are not intended to identify key or critical features of the embodiments of the present disclosure, nor limit the scope of the present disclosure. Other features of the present disclosure will become apparent from the following description.

**Brief Description of Drawings**

The drawings are used for better understanding the present solution and do not constitute a limitation of the present disclosure, wherein

Fig. 1 is a schematic diagram according to a first embodiment of the present disclosure;
Fig. 2 is a schematic diagram according to a second embodiment of the present disclosure;
Fig. 3 is a schematic diagram according to a third embodiment of the present disclosure;
Fig. 4 is a schematic diagram according to a fourth embodiment of the present disclosure;
Fig. 5 is a schematic diagram according to a fifth embodiment of the present disclosure;
Fig. 6 is a schematic diagram according to a sixth embodiment of the present disclosure;
Fig. 7 is a schematic diagram according to a seventh embodiment of the present disclosure;
Fig. 8 is a schematic diagram according to an eighth embodiment of the present disclosure;
Fig. 9 is a schematic diagram according to a ninth embodiment of the present disclosure; and
Fig. 10 shows a schematic block diagram of an exemplary electronic device 1000 configured to implement the embodiments of the present disclosure.

**Detailed Description of Preferred Embodiments**

The following part will illustrate exemplary embodiments of the present disclosure with reference to the drawings, including various details of the embodiments of the present disclosure for a better understanding. The embodiments should be regarded only as exemplary ones. Therefore, those skilled in the art should appreciate that various changes or modifications can be made with respect to the embodiments described herein without departing from the scope and spirit of the present disclosure. Similarly, for clarity and conciseness, the descriptions of the known functions and structures are omitted in the descriptions below.

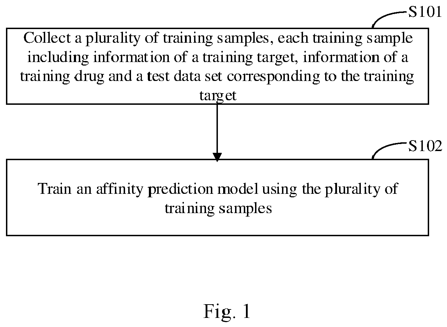

Fig. 1 is a schematic diagram according to a first embodiment of the present disclosure; as shown in Fig. 1, the present embodiment provides a method for training an affinity prediction model, which may include the following steps: S101: collecting a plurality of training samples, each training sample including information of a training target, information of a training drug and a test data set corresponding to the training target.

Each training sample may include the information of one training target, the information of one training drug and the test data set corresponding to this training target.

S102: training an affinity prediction model using the plurality of training samples.

An apparatus for training an affinity prediction model serves as the subject for executing the method for training an affinity prediction model according to the present embodiment, and may be configured as an electronic entity or a software-integrated application. In use, the affinity prediction model may be trained based on a plurality of training samples collected in advance.

Specifically, a number of the plural training samples collected in the present embodiment may reach an order of millions and above, and the greater the number of the collected training samples, the higher the accuracy of the trained affinity prediction model.

In the present embodiment, the plurality of collected training samples involve a plurality of training target samples, which means that part of the plurality of training samples may have same or different training targets. For example, one hundred thousand training targets may be involved in one million training samples, such that training samples with the same training targets inevitably exist in the one million training samples, but the training samples with the same training targets only mean that these training samples have the same training targets and different training drugs.

Unlike training data of a conventional model training operation, in the present embodiment, the training sample is required to include, in addition to the information of the training target and the information of the training drug, the test data set corresponding to the training target, so as to further improve a training effect of the affinity prediction model. For example, in the present embodiment, the test data set corresponding to the training target may include a known affinity of the training target with each tested drug for use in training the affinity prediction model. The information of the training target in the training sample may be an identifier of the training target, which is used to uniquely identify the training target, or may be an expression means of a protein of the training target. The information of the training drug in the training sample may be a molecular formula of a compound of the training drug or other identifier capable of uniquely identifying the training compound.

For example, in the present embodiment, the test data set corresponding to the training target may include plural pieces of test data, and a representation form of each piece of test data may be (the information of the training target, information of the tested drug, and an affinity between the training target and the tested drug). There may be existing a separate test data set for each training target to record the information of all the tested drugs on the training target.

The test data set corresponding to each training target is a special known data set, and the included affinity between the training target and each of a plurality of tested drugs, the information of the training target and the information of the training drug corresponding to the training target may form one training sample for use in the training operation of the affinity prediction model. Each training sample may include the information of one training target, the information of one training drug and the test data set corresponding to this training target.

Finally, the affinity prediction model is trained based on the plurality of training samples obtained in the above-mentioned way.

In the method for training an affinity prediction model according to the present embodiment, the plurality of training samples are collected, each training sample includes the information of the training target, the information of the training drug and the test data set corresponding to the training target; and the affinity prediction model is trained using the plurality of training samples; in the technical solution of the present embodiment, the test data set corresponding to the training target is added in each training sample, thus effectively improving the accuracy and the training effect of the trained affinity prediction model.

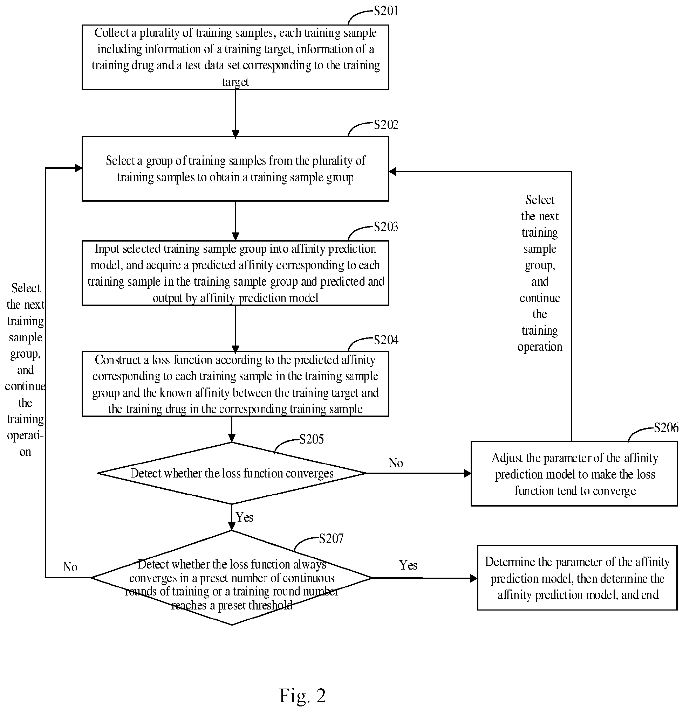

FIG. 2 is a schematic diagram according to a second embodiment of the present disclosure; as shown in Fig. 2, the technical solution of the method for training an affinity prediction model according to the present embodiment of the present disclosure is further described in more detail based on the technical solution of the above-mentioned embodiment shown in Fig. 1. As shown in Fig. 2, the method for training an affinity prediction model according to the present embodiment may include the following steps:

S201: collecting a plurality of training samples, each training sample including information of a training target, information of a training drug and a test data set corresponding to the training target.

For example, when plural training targets are involved in the plural training samples, each training target may be represented by $t_j$, and the test data set $D_{t_j}$ of the training target $t_j$ may be represented as:

$$\mathcal{D}_{t_j} = \left\{ \left( c_{j_1}, t_j, y\left( c_{j_1}, t_j \right) \right), \left( c_{j_2}, t_j, y\left( c_{j_2}, t_j \right) \right), \dots \right\}.$$

Each of $(c_{j_1}, t_j, y(c_{j_1}, t_j))$ and $(c_{j_2}, t_j, y(c_{j_2}, t_j))$ corresponds to one piece of test data, $c_{j_1}$ and $c_{j_2}$ are information of a tested drug and used for identifying the corresponding tested drug, and $t_j$ is the information of the training target and used for identifying the corresponding training target. $y(c_{j_1}, t_j)$ represents a known affinity between the tested drug $c_{j_1}$ and the training target $t_j$, and $y(c_{j_2}, t_j)$ represents a known affinity between the tested drug $c_{j_2}$ and the training target $t_j$. In the present embodiment, the known affinity may be detected experimentally. The test data set $D_{t_j}$ of the training target $t_j$ may include test data of all tested drugs corresponding to the training target $t_j$. In the present embodiment, the information of the training drug in the training sample may be represented by $c_i$.

S202: selecting a group of training samples from the plurality of training samples to obtain a training sample group.

For example, in practical applications, a group of training samples may be randomly selected from the plurality of training samples as a training sample group. Specifically, the training sample group may include one, two, or more training samples, which is not limited herein. If the training sample group includes more than two training samples, the training samples in the training sample group may correspond to the same training target, or some training samples may correspond to the same training target, and each of the other training samples corresponds to one training target.

S203: inputting the selected training sample group into the affinity prediction model, and acquiring a predicted affinity corresponding to each training sample in the training sample group and predicted and output by the affinity prediction model.

In the present embodiment, the affinity prediction model may be represented as:

$$y(c_i, t_j) = f\left( \mathcal{D}_{t_j}, c_i, t_j; \theta \right)$$

wherein $t_j$ represents the information of the training target, $c_i$ represents the information of the training drug, $D_{t_j}$ represents the test data set of the training target $t_j$, $\theta$ represents a parameter of the affinity prediction model, $f(D_{t_j}, c_i, t_j; \theta)$ represents the affinity prediction model, and $y(c_i, t_j)$ represents the affinity between the training target $t_j$ and the training drug $c_i$ predicted by the affinity prediction model.

For each training sample in the training sample group, an affinity prediction model may be acquired using the above-mentioned way, and a predicted affinity is predicted and output.

S204: constructing a loss function according to the predicted affinity corresponding to each training sample in the training sample group and the known affinity between the training target and the training drug in the corresponding training sample.

For example, if the training sample group includes only one training sample, a mean square error between the predicted affinity corresponding to the training sample and the corresponding known affinity is taken directly. The predicted affinity corresponding to the training sample means that the data in the training sample is input into the affinity prediction model, and the affinity between the training target $t_j$ and the training drug $c_i$ in the training sample is predicted by the affinity prediction model. The known affinity corresponding to the training sample may be an actual affinity obtained by experiments between the training target and the training drug in the test data set corresponding to the training target.

If the training sample group includes plural training samples, a sum of mean square errors between the predicted affinities corresponding to the training samples in the training sample group and the corresponding known affinities may be taken as the loss function. The present embodiment has a training purpose of making the loss function tend to converge to a minimum value, which, for example, may be represented by the following formula:

$$minimize\ \mathcal{L}(\theta) = \sum_{c_i, t_j, y_{ij}} \left[ y(c_i, t_j) - f\left( \mathcal{D}_{t_j}, c_i, t_j; \theta \right) \right]^2$$

S205: detecting whether the loss function converges; if no, executing step S206; and if yes, executing step S207.

S206: adjusting the parameter of the affinity prediction model to make the loss function tend to converge; and returning to step S202, selecting the next training sample group, and continuing the training operation.

S207: detecting whether the loss function always converges in a preset number of continuous rounds of training or whether a training round number reaches a preset threshold; if yes, determining the parameter of the affinity prediction model, then determining the affinity prediction model, and ending; otherwise, returning to step S202, selecting the next training sample group, and continuing the training operation.

Steps S202-S206 show the training process for the affinity prediction model. Step S207 is a training ending condition for the affinity prediction model. In the present embodiment, for example, the training ending condition has two cases; in the first training ending condition, whether the loss function always converges in the preset number of continuous rounds of training is determined, and if the loss function always converges, it may be considered that the training operation of the affinity prediction model is completed. The preset number of the continuous rounds may be set according to actual requirements, and may be, for example, 80, 100, 200 or other positive integers, which is not limited herein. The second training ending condition prevents a situation that the loss function always tends to converge, but never reaches convergence. At this point, a maximum number of training rounds may be set, and when the number of training rounds reaches the maximum number of training rounds, it may be considered that the training operation of the affinity prediction model is completed. For example, the preset threshold may be set to a value on the order of millions or above according to actual requirements, which is not limited herein.

In the present embodiment, the more the test data included in the test data set on each training target, the better the prediction effect achieved by the affinity prediction model. To this end, in the present disclosure, an attention layer model for processing a sequence may be used to obtain an optimal effect. For example, the model may be represented as follows:

$$Attention(Q, K, V) = Softmax(\frac{QK^T}{\alpha})V$$

The target may be represented and labeled as $\phi(t_j)$, a drug molecule may be represented and labeled as $\phi(c_i)$, and fusion of the two representations may be labeled as $\phi(c_i, t_j)$.

$$Q = \phi(c_i, t_j)$$
,

and

$$K = V = \{(\phi(c_{i_1}, t_j), y(c_{i_2}, t_j)), (\phi(c_{i_2}, t_j), y(c_{i_2}, t_j)), ...\}$$
,

such that a pair to be predicted may be fully extracted using the existing information of the target. A predicted form of the final model may be represented as:

$$f(\mathcal{D}_{t_j}, c_i, t_j; \theta) = MLP(Attention(Q, K, V))$$

wherein *MLP(Attention(Q, K, V))* indicates that a model structure *Attention(Q, K, V)* may be adjusted.

In addition, it should be noted that the affinity prediction model in the present embodiment is not limited to the above-mentioned attention layer model, and a Transformer model or a convolution neural network model, or the like, may also be used, which is not repeated herein.

In the method for training an affinity prediction model according to the present embodiment, the test data set corresponding to the training target may be added in each training sample, thus effectively improving the accuracy and the training effect of the trained affinity prediction model.

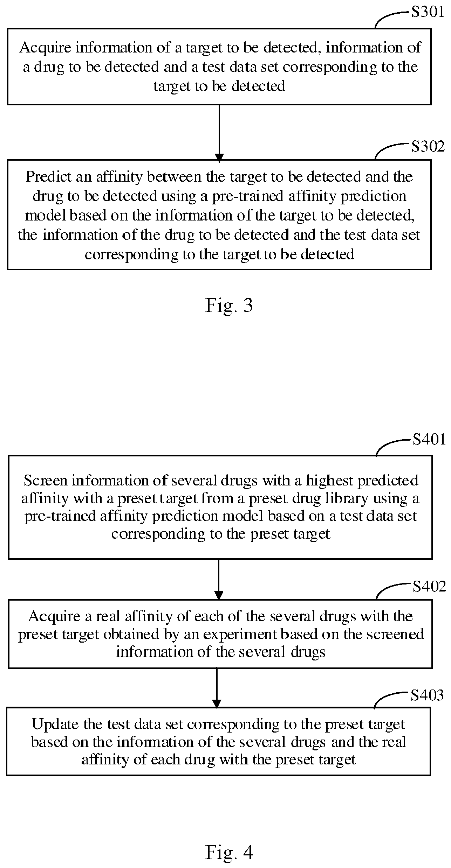

Fig. 3 is a schematic diagram according to a third embodiment of the present disclosure; as shown in Fig. 3, the present embodiment provides an affinity prediction method, which may include the following steps:

S301: acquiring information of a target to be detected, information of a drug to be detected and a test data set corresponding to the target to be detected.

In the present embodiment, the test data set includes information of one target to be detected, information of a plurality of tested drugs and an affinity between the target to be detected and each tested drug. For details, reference may be made to the test data set in the above-mentioned embodiment shown in Fig. 1 or Fig. 2.

S302: predicting an affinity between the target to be detected and the drug to be detected using a pre-trained affinity prediction model based on the information of the target to be detected, the information of the drug to be detected and the test data set corresponding to the target to be detected.

An affinity prediction apparatus serves as the subject for executing the affinity prediction method according to the present embodiment, and similarly, may be configured as an electronic entity or a software-integrated application. In use, the target to be detected, the drug to be detected and the test data set corresponding to the target to be detected may be input into the affinity prediction apparatus, and the affinity prediction apparatus may predict and output the affinity between the target to be detected and the drug to be detected based on the input information.

In the present embodiment, the adopted pre-trained affinity prediction model may be the affinity prediction model trained in the embodiment shown in Fig. 1 or Fig. 2. And since the test data set of the training target is added into the training sample in the training process, the trained affinity prediction model may have higher precision and better accuracy. Therefore, the thus trained affinity prediction model may effectively guarantee the quite high precision and the quite good accuracy of the predicted affinity between the target to be detected and the drug to be detected.

In the present embodiment, the higher the predicted affinity between the target to be detected and the drug to be detected is, the stronger the binding capacity between the target to be detected and the drug to be detected is, the higher the inhibition of the target to be detected by the drug to be detected is, and the more likely the drug to be detected is to become an effective therapeutic drug for the target to be detected.

In the affinity prediction method according to the present embodiment, the target to be detected, the drug to be detected and the test data set corresponding to the target to be detected are acquired; the affinity between the target to be detected and the drug to be detected is predicted using the pre-trained affinity prediction model based on the target to be detected, the drug to be detected and the test data set corresponding to the target to be detected; since the test data set corresponding to the target to be detected is acquired during the prediction to participate in the prediction, the predicted affinity between the target to be detected and the drug to be detected may have higher accuracy.

Fig. 4 is a schematic diagram according to a fourth embodiment of the present disclosure; as shown in Fig. 4, the present embodiment provides a method for screening drug data, which may include the following steps:

S401: screening information of several drugs with a highest predicted affinity with a preset target from a preset drug library using a pre-trained affinity prediction model based on a test data set corresponding to the preset target;

S402: acquiring a real affinity of each of the several drugs with the preset target obtained by an experiment based on the screened information of the several drugs; and

S403: updating the test data set corresponding to the preset target based on the information of the several drugs and the real affinity of each drug with the preset target.

An apparatus for screening drug data serves as the subject for executing the method for screening drug data according to the present embodiment, and the apparatus for screening drug data may screen the several drugs with the highest predicted affinity of each preset target and update the drugs to the corresponding test data set.

In the present embodiment, the pre-trained affinity prediction model may be the affinity prediction model trained using the training method according to the above-mentioned embodiment shown in Fig. 1 or Fig. 2. That is, the test data set of the training target is added into the training sample in the training process, such that the trained affinity prediction model may have higher precision and better accuracy.

In the present embodiment, for example, the drug of one preset target is screened, and the test data set of the preset target is updated; the test data set of the preset target may be acquired, reference may be made to relevant descriptions in the above-mentioned embodiment for data included in the test data set, and the data is not repeated here.

The preset drug library in the present embodiment may include information of thousands or even more of drugs which are not verified experimentally, such as molecular formulas of compounds of the drugs or other unique identification information of the drugs. If the affinity between each drug in the drug library and the preset target is directly verified using an experimental method, an experimental cost is quite high. In the present embodiment, first, the information of the several drugs with the highest predicted affinity with the preset target may be screened from the preset drug library using the pre-trained affinity prediction model based on the test data set corresponding to the preset target; the number of the several drugs may be set according to actual requirements, and may be, for example, 5, 8, 10, or other positive integers, which is not limited herein. The screening operation in step S401 is performed by the affinity prediction model, these drugs have high predicted affinities with the preset target, and availability of these drugs is theoretically high under a condition that the trained affinity prediction model performs an accurate prediction. Therefore, the known affinities between the screened drugs and the preset target may be further detected experimentally, thus avoiding experimentally detecting every drug in the drug library, so as to reduce the experimental cost and improve a drug screening efficiency. Then, the information of the several experimentally detected drugs and the real affinity of each drug with the preset target are updated into the test data set corresponding to the preset target, so as to complete one screening operation.

In the present embodiment, the information of the several drugs and the real affinity of each drug with the preset target are updated into the test data set corresponding to the preset target, thus enriching content of test data in the test data set, such that the screening efficiency may be improved when the next screening operation is performed based on the test data set.

In the drug processing method according to the present embodiment, with the above-mentioned solution, the information of the several drugs with the highest predicted affinity with the preset target may be screened from the preset drug library using the pre-trained affinity prediction model based on the test data set corresponding to the preset target, and then, the real affinity of each of the several screened drugs with the preset target is detected using the experimental method; the information of the several drugs and the real affinity of each drug with the preset target are updated into the test data set corresponding to the preset target, thus effectively avoiding experimentally screening all the drugs, so as to reduce the experimental cost and improve the drug screening efficiency.

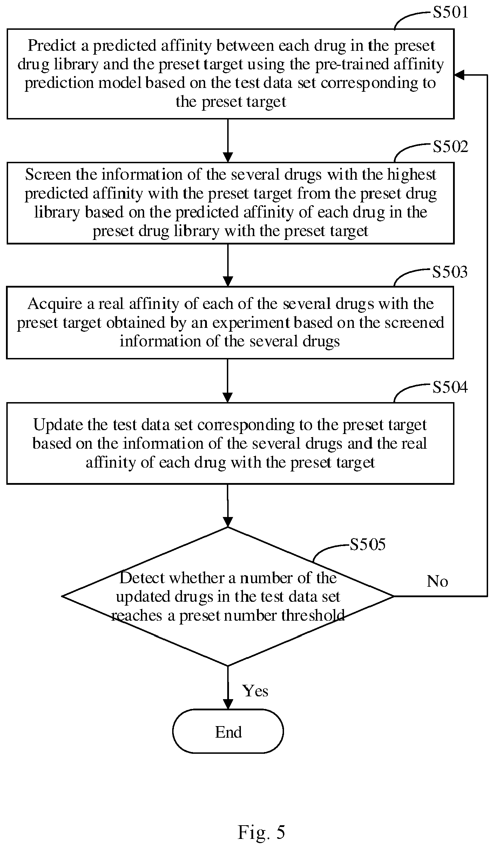

Fig. 5 is a schematic diagram according to a fifth embodiment of the present disclosure; as shown in Fig. 5, the technical solution of the method for screening drug data according to the present embodiment of the present application is further described in more detail based on the technical solution of the above-mentioned embodiment shown in Fig. 4. The method for screening drug data according to the present embodiment may specifically include the following steps: S501: predicting a predicted affinity between each drug in the preset drug library and the preset target using the pre-trained affinity prediction model based on the test data set corresponding to the preset target.

It should be noted that during the first prediction, the test data set corresponding to the preset target may also be null. For example, for a preset target t and a drug library $C = \{c_1, ..., c_M\}$, at the current step number s=1, i.e., at the beginning of a cycle, a test data set $\mathcal{D}_t$ corresponding to the preset target may be represented as $\mathcal{D}_t = \{\}$. Certainly, during the first prediction, the test data set corresponding to the preset target may not be null, and includes the preset target, information of an experimentally verified drug, and the known affinity between the preset target and the drug. At this point, the amount of the relevant information of the drug included in the test data set corresponding to the preset target is not limited herein.

S502: screening the information of the several drugs with the highest predicted affinity with the preset target from the preset drug library based on the predicted affinity of each drug in the preset drug library with the preset target.

The steps S501-S502 are an implementation of the above-mentioned embodiment shown in Fig. 4. That is, the information of each drug in the preset drug library, the information of the preset target, and the test data set of the preset target are input into the pre-trained affinity prediction model, and the affinity prediction model may predict and output the predicted affinity between the drug and the preset target. In this way, the predicted affinity between each drug in the drug library and the preset target may be predicted. Then, all the drugs in the preset drug library may be sequenced according to a descending order of the predicted affinity; and the several drugs with the highest predicted affinity may be screened.

S503: acquiring a real affinity of each of the several drugs with the preset target obtained by an experiment based on the screened information of the several drugs; and

In the present embodiment, only the several drugs screened in step S502 are required to be experimented to obtain the real affinity between each of the several drugs and the preset target. For example, $C_{s_i}$ may be used to represent information of the screened ith drug, $i \in [1, K]$, and K represents the number of the several drugs. Correspondingly, $y(C_{s_i}, t)$ is used to represent the real affinity of the screened ith drug with the preset target t.

S504: updating the test data set corresponding to the preset target based on the information of the several drugs and the real affinity of each drug with the preset target.

For example, the update process may be represented by the following formula:

$$\mathcal{D}_t = \mathcal{D}_t \cup \left\{ \left( c_{s_1}, y\left( c_{s_1}, t \right) \right), ..., \left( c_{s_K}, y\left( c_{s_K}, t \right) \right) \right\}$$

S505: detecting whether a number of the updated drugs in the test data set reaches a preset number threshold; if no, returning to step S501 to continuously screen the drugs; otherwise, if yes, ending.

It should be noted that, in the present embodiment, the number of the updated drugs in the test data set may refer to a number of the drugs with the known affinities acquired experimentally. At the first update, the number of the drugs updated into the test data set may be the number of all the screened drugs. In other rounds of updates after the cycle, since repetition may exist between the information of the several screened drugs and the previous information, the number of the updated drugs in the test data set may be less than the number of the screened drugs.

In the present embodiment, if the number of the experimented drugs does not reach the preset number threshold, the method may return to step S501, the current step number s is updated to s+1, and the screening operation is continuously performed. Although the same pre-trained affinity prediction model is adopted in the second screening process, the adopted test data set of the preset target is updated, thereby further improving the accuracy of the affinity of each drug in the drug library with the preset target. Therefore, when the second screening process is performed based on the updated test data set of the preset target, the information of the several drugs with the highest predicted affinity with the preset target screened from the preset drug library may be completely different from or partially the same as the result of the several drugs screened in the previous round. It should be noted that, in the partially same case, in step S503, for the experimented drugs, experiments may not be performed again to obtain the rear affinities with the predetermined target. Only the drugs which are not experimented are experimented to obtain the real affinities with the preset target, and only the real affinities of the drugs obtained by experiments in this round with the preset target of the drugs are updated in the test data set, and so on, until the number of the updated drugs in the test data set reaches the preset number threshold, and the cycle is ended. At this point, the data in the test data set is all the real affinities with the preset target obtained through experiments. Subsequently, the information of one or several drugs with the highest known affinity may be selected from the test data set of the preset target, and the selected drugs may be used as lead drug compounds for subsequent verification.

The test data set corresponding to the preset target obtained by the screening operation in the present embodiment may also be used in the training process of the affinity prediction model in the embodiment shown in Fig. 1 or Fig. 2, thus effectively guaranteeing the accuracy of the test data set of the preset target in the training sample, and then further improving the precision of the trained affinity prediction model. In turn, the affinity prediction model in the embodiment shown in Fig. 1 or Fig. 2 is used to screen the drug data in the embodiment shown in Fig. 4 or Fig. 5, which may also improve the screening accuracy and the screening efficiency of the drug data.

Or the test data set corresponding to the preset target obtained by the screening operation in the present embodiment may also be different from the test data set in the training sample in the embodiment shown in Fig. 1 or Fig. 2. In the present embodiment, since the pre-trained affinity prediction model is first adopted to screen the information of the several drugs, in the test data set finally obtained based on the information of the several drugs, the preset target and the drugs have higher affinities; however, in the test data set in the training sample in the embodiment shown in Fig. 1 or Fig. 2, the training target and the test drug may have a low affinity, as long as it is obtained through experiments.

In the method for screening drug data according to the present embodiment, with the above-mentioned solution, the pre-trained affinity detection model may be utilized to provide an effective drug screening solution, thus avoiding experimentally screening all the drugs in the drug library, so as to effectively reduce the experimental cost and improve the drug screening efficiency.

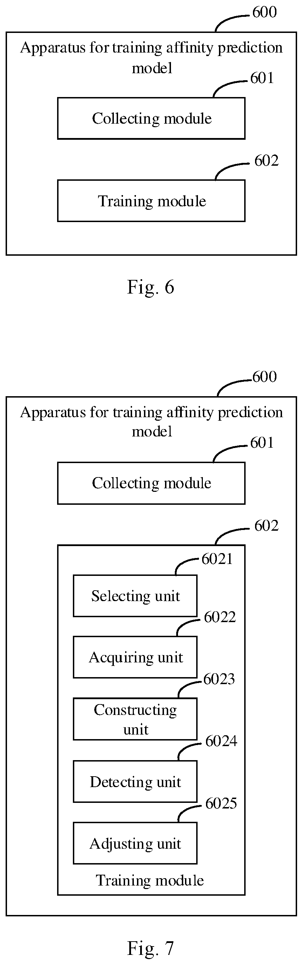

Fig. 6 is a schematic diagram according to a sixth embodiment of the present disclosure. As shown in Fig. 6, the present embodiment provides an apparatus for training an affinity prediction model, including:

> a collecting module 601 configured to collect a plurality of training samples, each training sample including information of a training target, information of a training drug and a test data set corresponding to the training target; and
> a training module 602 configured to train an affinity prediction model using the plurality of training samples.

The apparatus 600 for training an affinity prediction model according to the present embodiment has the same implementation as the above-mentioned relevant method embodiment by adopting the above-mentioned modules to implement the implementation principle and the technical effects of training the affinity prediction model, and for details, reference may be made to the description of the above-mentioned relevant method embodiment, and details are not repeated herein.

FIG. 7 is a schematic diagram according to a seventh embodiment of the present disclosure. As shown in Fig. 7, the technical solution of the apparatus 600 for training an affinity prediction model according to the present embodiment of the present application is further described in more detail based on the technical solution of the above-mentioned embodiment shown in Fig. 6.

In the apparatus 600 for training an affinity prediction model according to the present embodiment, the test data set corresponding to the training target in each of the plural training samples collected by the collecting module 601 may include a known affinity of the training target with each tested drug.

As shown in Fig. 7, in the apparatus 600 for training an affinity prediction model according to the present embodiment, the training module 602 includes:

> a selecting unit 6021 configured to select a group of training samples from the plurality of training samples to obtain a training sample group;

an acquiring unit 6022 configured to input the selected training sample group into the affinity prediction model, and acquire a predicted affinity corresponding to each training sample in the training sample group and predicted and output by the affinity prediction model;

a constructing unit 6023 configured to construct a loss function according to the predicted affinity corresponding to each training sample in the training sample group and the known affinity between the training target and the training drug in the corresponding training sample;

a detecting unit 6024 configured to detect whether the loss function converges; and

an adjusting unit 6025 configured to, if the loss function does not converge, adjust parameters of the affinity prediction model to make the loss function tend to converge.

Further optionally, the constructing unit 6023 is configured to:
take a sum of mean square errors between the predicted affinities corresponding to the training samples in the training sample group and the corresponding known affinities as the loss function.

The apparatus 600 for training an affinity prediction model according to the present embodiment has the same implementation as the above-mentioned relevant method embodiment by adopting the above-mentioned modules to implement the implementation principle and the technical effects of training the affinity prediction model, and for details, reference may be made to the description of the above-mentioned relevant method embodiment, and details are not repeated herein.

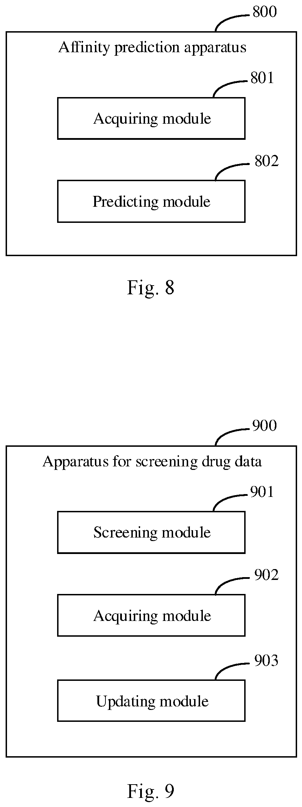

Fig. 8 is a schematic diagram according to an eighth embodiment of the present disclosure; as shown in Fig. 8, the present embodiment provides an affinity prediction apparatus 800, including:

an acquiring module 801 configured to acquire information of a target to be detected, information of a drug to be detected and a test data set corresponding to the target to be detected; and

a predicting module 802 configured to predict an affinity between the target to be detected and the drug to be detected using a pre-trained affinity prediction model based on the information of the target to be detected, the information of the drug to be detected and the test data set corresponding to the target to be detected.

The affinity prediction apparatus 800 according to the present embodiment has the same implementation as the above-mentioned relevant method embodiment by adopting the above-mentioned modules to implement the implementation principle and the technical effects of the affinity prediction, and for details, reference may be made to the description of the above-mentioned relevant method embodiment, and details are not repeated herein.

Fig. 9 is a schematic diagram according to a ninth embodiment of the present disclosure. As shown in Fig. 9, the present embodiment provides an apparatus 900 for screening drug data, including:

a screening module 901 configured to screen information of several drugs with a highest predicted affinity with a preset target from a preset drug library using a pre-trained affinity prediction model based on a test data set corresponding to the preset target;

an acquiring module 902 configured to acquire a real affinity of each of the several drugs with the preset target obtained by an experiment based on the screened information of the several drugs; and

an updating module 903 configured to update the test data set corresponding to the preset target based on the information of the several drugs and the real affinity of each drug with the preset target.

The apparatus 900 for screening drug data according to the present embodiment has the same implementation as the above-mentioned relevant method embodiment by adopting the above-mentioned modules to implement the implementation principle and the technical effects of screening drug data, and for details, reference may be made to the description of the above-mentioned relevant method embodiment, and details are not repeated herein.

The present disclosure further provides an electronic device, a readable storage medium and a computer program product.

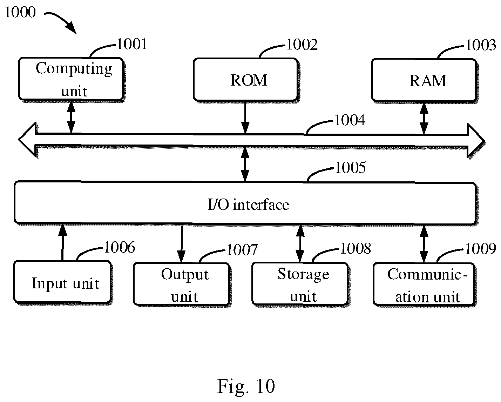

Fig. 10 shows a schematic block diagram of an exemplary electronic device 1000 configured to implement the embodiments of the present disclosure. The electronic device is intended to represent various forms of digital computers, such as laptop computers, desktop computers, workstations, servers, blade servers, mainframe computers, and other appropriate computers. The electronic device may also represent various forms of mobile apparatuses, such as personal digital assistants, cellular telephones, smart phones, wearable devices, and other similar computing apparatuses. The components shown herein, their connections and relationships, and their functions, are meant to be exemplary only, and are not meant to limit implementation of the present disclosure described and/or claimed herein.

As shown in Fig. 10, the electronic device 1000 includes a computing unit 1001 which may perform various appropriate actions and processing operations according to a computer program stored in a read only memory (ROM) 1002 or a computer program loaded from a storage unit 1008 into a random access memory (RAM) 1003. Various programs and data necessary for the operation of the electronic device 1000 may be also stored in the RAM 1003. The computing unit 1001, the ROM 1002, and the RAM 1003 are connected with one other through a bus 1004. An input/output (I/O) interface 1005 is also connected to the bus 1004.

The plural components in the electronic device 1000 are connected to the I/O interface 1005, and include: an input unit 1006, such as a keyboard, a mouse, or the like; an output unit 1007, such as various types of displays, speakers, or the like; the storage unit 1008, such as a magnetic disk, an optical disk, or the like; and a communication unit 1009, such as a network card, a modem, a wireless communication transceiver, or the like. The communication unit 1009 allows the electronic device 1000 to exchange information/data with other devices through a computer network, such as the Internet, and/or various telecommunication networks.

The computing unit 1001 may be a variety of general and/or special purpose processing components with processing and computing capabilities. Some examples of the computing unit 1001 include, but are not limited to, a central processing unit (CPU), a graphic processing unit (GPU), various dedicated artificial intelligence (AI) computing chips, various computing units running machine learning model algorithms, a digital signal processor (DSP), and any suitable processor, controller, microcontroller, or the like. The computing unit 1001 performs the methods and processing operations described above, such as the method for training an affinity prediction model, the affinity prediction method or the method for screening drug data. For example, in some embodiments, the method for training an affinity prediction model, the affinity prediction method or the method for screening drug data may be implemented as a computer software program tangibly contained in a machine readable medium, such as the storage unit 1008. In some embodiments, part or all of the computer program may be loaded and/or installed into the electronic device 1000 via the ROM 1002 and/or the communication unit 1009. When the computer program is loaded into the RAM 1003 and executed by the computing unit 1001, one or more steps of the method for training an affinity prediction model, the affinity prediction method or the method for screening drug data described above may be performed. Alternatively, in other embodiments, the computing unit 1001 may be configured to perform the method for training an affinity prediction model, the affinity prediction method or the method for screening drug data by any other suitable means (for example, by means of firmware).

Various implementations of the systems and technologies described herein above may be implemented in digital electronic circuitry, integrated circuitry, field programmable gate arrays (FPGA), application specific integrated circuits (ASIC), application specific standard products (ASSP), systems on chips (SOC), complex programmable logic devices (CPLD), computer hardware, firmware, software, and/or combinations thereof. The systems and technologies may be implemented in one or more computer programs which are executable and/or interpretable on a programmable system including at least one programmable processor, and the programmable processor may be special or general, and may receive data and instructions from, and transmit data and instructions to, a storage system, at least one input apparatus, and at least one output apparatus.

Program codes for implementing the method according to the present disclosure may be written in any combination of one or more programming languages. These program codes may be provided to a processor or a controller of a general purpose computer, a special purpose computer, or other programmable data processing apparatuses, such that the program code, when executed by the processor or the controller, causes functions/operations specified in the flowchart and/or the block diagram to be implemented. The program code may be executed entirely on a machine, partly on a machine, partly on a machine as a stand-alone software package and partly on a remote machine, or entirely on a remote machine or a server.

In the context of the present disclosure, the machine readable medium may be a tangible medium which may contain or store a program for use by or in connection with an instruction execution system, apparatus, or device. The machine readable medium may be a machine readable signal medium or a machine readable storage medium. The machine readable medium may include, but is not limited to, an electronic, magnetic, optical, electromagnetic, infrared, or semiconductor system, apparatus, or device, or any suitable combination of the foregoing. More specific examples of the machine readable storage medium may include an electrical connection based on one or more wires, a portable computer disk, a hard disk, a random access memory (RAM), a read only memory (ROM), an erasable programmable read only memory (EPROM or flash memory), an optical fiber, a portable compact disc read only memory (CD-ROM), an optical storage device, a magnetic storage device, or any suitable combination of the foregoing.

To provide interaction with a user, the systems and technologies described here may be implemented on a computer having: a display apparatus (for example, a cathode ray tube (CRT) or liquid crystal display (LCD) monitor) for displaying information to a user; and a keyboard and a pointing apparatus (for example, a mouse or a trackball) by which a user may provide input for the computer. Other kinds of apparatuses may also be used to provide interaction with a user; for example, feedback provided for a user may be any form of sensory feedback (for example, visual feedback, auditory feedback, or tactile feedback); and input from a user may be received in any form (including acoustic, speech or tactile input).

The systems and technologies described here may be implemented in a computing system (for example, as a data server) which includes a back-end component, or a computing system (for example, an application server) which includes a middleware component, or a computing system (for example, a user computer having a graphical user interface or a web browser through which a user may interact with an implementation of the systems and technologies described here) which includes a front-end component, or a computing system which includes any combination of such back-end, middleware, or front-end components. The components of the system may be interconnected through any form or medium of digital data communication (for example, a communication network). Examples of the communication network include: a local area network (LAN), a wide area network (WAN), the Internet and a blockchain network.

A computer system may include a client and a server. Generally, the client and the server are remote from each other and interact through the communication network. The relationship between the client and the server is generated by virtue of computer programs which run on respective computers and have a client-server relationship to each other. The server may be a cloud server, also called a cloud computing server or a cloud host, and is a host product in a cloud computing service system, so as to overcome the defects of high management difficulty and weak service expansibility in conventional physical host and virtual private server (VPS) service. The server may also be a server of a distributed system, or a server incorporating a blockchain.

It should be understood that various forms of the flows shown above may be used and reordered, and steps may be added or deleted. For example, the steps described in the present disclosure may be executed in parallel, sequentially, or in different orders, which is not limited herein as long as the desired results of the technical solution disclosed in the present disclosure may be achieved.

The above-mentioned implementations are not intended to limit the scope of the present disclosure. It should be understood by those skilled in the art that various modifications, combinations, sub-combinations and substitutions may be made, depending on design requirements and other factors. Any modification, equivalent substitution and improvement made within the spirit and principle of the present disclosure all should be included in the extent of protection of the present disclosure.

**Claims**

1. A method for training an affinity prediction model, comprising:

   collecting (S101) a plurality of training samples, each training sample comprising information of a training target, information of a training drug and a test data set corresponding to the training target; and
   training (S102) an affinity prediction model using the plurality of training samples.

2. The method according to claim 1, wherein the test data set corresponding to the training target comprises a known affinity of the training target with each tested drug.

3. The method according to claim 2, wherein the training an affinity prediction model using the plurality of training samples comprises:

   selecting (S202) a group of training samples from the plurality of training samples to obtain a training sample group;
   inputting (S203) the selected training sample group into the affinity prediction model, and acquiring (S203) a predicted affinity corresponding to each training sample in the training sample group and predicted and output by the affinity prediction model;
   constructing (S204) a loss function according to the predicted affinity corresponding to each training sample in the training sample group and the known affinity between the training target and the training drug in the corresponding training sample;
   detecting (S205) whether the loss function converges; and
   if the loss function does not converge, adjusting (S206) parameters of the affinity prediction model to make the loss function tend to converge.

4. The method according to claim 3, wherein the constructing a loss function according to the predicted affinity corresponding to each training sample in the training sample group and the known affinity between the training target and the training drug in the corresponding training sample comprises:
   taking a sum of mean square errors between the predicted affinities corresponding to the training samples in the training sample group and the corresponding known affinities as the loss function.

5. An affinity prediction method, comprising:

   acquiring (S301) information of a target to be detected, information of a drug to be detected and a test data set corresponding to the target to be detected; and

predicting (S302) an affinity between the target to be detected and the drug to be detected using a pre-trained affinity prediction model based on the information of the target to be detected, the information of the drug to be detected and the test data set corresponding to the target to be detected.

6. The method according to claim 5, wherein the test data set corresponding to the target to be detected comprises the information of the target to be detected, information of a plurality of tested drugs and an affinity between the target to be detected and each of the plural tested drugs.

7. A method for screening drug data, comprising:

screening (S401) information of several drugs with a highest predicted affinity with a preset target from a preset drug library using a pre-trained affinity prediction model based on a test data set corresponding to the preset target;

acquiring (S402) a real affinity of each of the several drugs with the preset target based on the screened information of the several drugs; and

updating (S403) the test data set corresponding to the preset target based on the information of the several drugs and the real affinity of each drug with the preset target.

8. The method according to claim 7, wherein the test data set corresponding to the preset target is null or comprises information of a drug and a real affinity of the drug with the preset target.

9. The method according to claim 7 or 8, wherein the screening information of several drugs with a highest predicted affinity with a preset target from a preset drug library using a pre-trained affinity prediction model based on a test data set corresponding to the preset target comprises:

predicting a predicted affinity between each drug in the preset drug library and the preset target using the pre-trained affinity prediction model based on the test data set corresponding to the preset target; and

screening the information of the several drugs with the highest predicted affinity with the preset target from the preset drug library based on the predicted affinity of each drug in the preset drug library with the preset target.

10. An apparatus (600) for training an affinity prediction model, comprising:

a collecting module (601) configured to collect a plurality of training samples, each training sample comprising information of one training target, information of a training drug and a test data set corresponding to the training target; and

a training module (602) configured to train an affinity prediction model using the plurality of training samples.

11. An affinity prediction apparatus (800), comprising:

an acquiring module (801) configured to acquire information of a target to be detected, information of a drug to be detected and a test data set corresponding to the target to be detected; and

a predicting module (802) configured to predict an affinity between the target to be detected and the drug to be detected using a pre-trained affinity prediction model based on the information of the target to be detected, the information of the drug to be detected and the test data set corresponding to the target to be detected.

12. An apparatus (900) for screening drug data, comprising:

a screening module (901) configured to screen information of several drugs with a highest predicted affinity with a preset target from a preset drug library using a pre-trained affinity prediction model based on a test data set corresponding to the preset target;

an acquiring module (902) configured to acquire a real affinity of each of the several drugs with the preset target obtained by an experiment based on the screened information of the several drugs; and

an updating module (903) configured to update the test data set corresponding to the preset target based on the information of the several drugs and the real affinity of each drug with the preset target.

13. An electronic device, comprising:

at least one processor; and
a memory connected with the at least one processor communicatively;
wherein the memory stores instructions executable by the at least one processor to enable the at least one processor to perform the method according to any one of claims 1 to 4, 5 to 6 or 7 to 9.

14. A non-transitory computer readable storage medium with computer instructions stored thereon, wherein the computer instructions are used for causing a computer to perform the method according to any one of claims 1 to 4, 5 to 6 or 7 to 9.

15. A computer program product comprising a computer program which, when executed by a processor, implements the method according to any one of claims 1 to 4, 5 to 6 or 7 to 9.

S101

Collect a plurality of training samples, each training sample including information of a training target, information of a training drug and a test data set corresponding to the training target

S102

Train an affinity prediction model using the plurality of training samples

Fig. 1

S201

Collect a plurality of training samples, each training sample including information of a training target, information of a training drug and a test data set corresponding to the training target

S202

Select a group of training samples from the plurality of training samples to obtain a training sample group

Select the next training sample group, and continue the training operation

S203

Input selected training sample group into affinity prediction model, and acquire a predicted affinity corresponding to each training sample in the training sample group and predicted and output by affinity prediction model

Select the next training sample group, and continue the training operation

S204

Construct a loss function according to the predicted affinity corresponding to each training sample in the training sample group and the known affinity between the training target and the training drug in the corresponding training sample

S205

Detect whether the loss function converges — No →

S206

Adjust the parameter of the affinity prediction model to make the loss function tend to converge

Yes

S207

Detect whether the loss function always converges in a preset number of continuous rounds of training or a training round number reaches a preset threshold

No

Yes

Determine the parameter of the affinity prediction model, then determine the affinity prediction model, and end

Fig. 2

S301

Acquire information of a target to be detected, information of a drug to be detected and a test data set corresponding to the target to be detected

S302

Predict an affinity between the target to be detected and the drug to be detected using a pre-trained affinity prediction model based on the information of the target to be detected, the information of the drug to be detected and the test data set corresponding to the target to be detected

Fig. 3

S401

Screen information of several drugs with a highest predicted affinity with a preset target from a preset drug library using a pre-trained affinity prediction model based on a test data set corresponding to the preset target

S402

Acquire a real affinity of each of the several drugs with the preset target obtained by an experiment based on the screened information of the several drugs

S403

Update the test data set corresponding to the preset target based on the information of the several drugs and the real affinity of each drug with the preset target

Fig. 4

S501

Predict a predicted affinity between each drug in the preset drug library and the preset target using the pre-trained affinity prediction model based on the test data set corresponding to the preset target

S502

Screen the information of the several drugs with the highest predicted affinity with the preset target from the preset drug library based on the predicted affinity of each drug in the preset drug library with the preset target

S503

Acquire a real affinity of each of the several drugs with the preset target obtained by an experiment based on the screened information of the several drugs

S504

Update the test data set corresponding to the preset target based on the information of the several drugs and the real affinity of each drug with the preset target

S505

Detect whether a number of the updated drugs in the test data set reaches a preset number threshold

No

Yes

End

Fig. 5

600

Apparatus for training affinity prediction model

601

Collecting module

602

Training module

Fig. 6

600

Apparatus for training affinity prediction model

601

Collecting module

602

6021

Selecting unit

6022

Acquiring unit

6023

Constructing unit

6024

Detecting unit

6025

Adjusting unit

Training module

Fig. 7

```
                                              ┌── 800
┌─────────────────────────────────────────────┐
│  Affinity prediction apparatus              │
│                                   ┌── 801    │
│   ┌──────────────────────────────┐          │
│   │      Acquiring module        │          │
│   └──────────────────────────────┘          │
│                                   ┌── 802    │
│   ┌──────────────────────────────┐          │
│   │      Predicting module       │          │
│   └──────────────────────────────┘          │
│                                              │
└─────────────────────────────────────────────┘
```

Fig. 8

```
                                              ┌── 900
┌─────────────────────────────────────────────┐
│  Apparatus for screening drug data          │
│                                   ┌── 901    │
│   ┌──────────────────────────────┐          │
│   │      Screening module        │          │
│   └──────────────────────────────┘          │
│                                   ┌── 902    │
│   ┌──────────────────────────────┐          │
│   │      Acquiring module        │          │
│   └──────────────────────────────┘          │
│                                   ┌── 903    │
│   ┌──────────────────────────────┐          │
│   │      Updating module         │          │
│   └──────────────────────────────┘          │
│                                              │
└─────────────────────────────────────────────┘
```

Fig. 9

Fig. 10